# EUROPEAN PATENT APPLICATION

(11) **EP 0 575 020 A1**
(43) Date of publication of application: **22.12.1993**
(21) Application number: 93250151.3
(22) Date of filing: 03.06.1993
(51) Int. Cl.: A61K 7/16, A61K 9/00

(54) **Non-aqueous liquid antibacterial oral composition exhibiting improved uptake on dental tissue surfaces**

(30) Priority: 19.06.1992 US 901635
(71) Applicant: Colgate-Palmolive Company, New York, N.Y. 10022-7499 (US)
(72) Inventor: Prencipe, Michael, East Windsor, New Jersey (US); Drago, Vincent O., Highland Park, New Jersey (US); Curtis, John P., Bloomsbury, New Jersey (US); Greenfeder, Susan, Woodbridge, New Jersey (US)
(74) Representative: UEXKÜLL & STOLBERG

(57) **Abstract**

A non-aqueous liquid oral composition which exhibits increased uptake by dental tissue of antibacterial compounds incorporated therein, the composition containing an antibacterial compound and a water insoluble biodegradable polymer.

## Description

The invention provides a non-aqueous liquid antibacterial oral composition such as a mouthwash, rinse or spray which includes a water insoluble antibacterial compound such as triclosan and a water insoluble biodegradable polymer such as poly (DL-lactide) whereby the uptake and adherence of the antibacterial compound on dental tissue is substantially increased due to the presence of the polymer.

### BACKGROUND OF THE INVENTION

### 1. Field of the Invention

The present invention relates to oral care compositions which are designed to improve the effectiveness of antibacterial compounds in the retardation and prevention of bacterial "plaque" accumulation on the teeth.

### 2. The Prior Art

Dental plaque is a soft deposit which forms on teeth and is comprised of an accumulation of bacteria and bacterial by-products. Plaque adheres tenaciously at the points of irregularity or discontinuity, e.g., on rough calculus surfaces, at the gum line and the like. Besides being unsightly, plaque is implicated in the occurrence of gingivitis and other forms of periodontal disease.

A wide variety of antibacterial agents have been suggested in the art to retard plaque formation and the oral infections and dental disease associated with plaque formation. For example, halogenated hydroxydiphenyl ether compounds such as triclosan are well known to the art for their antibacterial activity and have been used in aqueous based oral compositions to counter plaque formation by bacterial accumulation in the oral cavity.

Although antibacterial agents such as triclosan are highly effective in killing bacteria which are responsible for plaque formation, it is difficult to maintain an effective level of such agents on dental tissue for a significant time period after their application. Thus, once applied, it is important that the antibacterial compound be maintained in strong and continuing adherence to the teeth and adjacent oral gingival mucosa thereby retarding washout of the antibacterial compound from infected areas of dental tissue by saliva present in the mouth. This allows for a sufficient amount of compound to remain in contact with the dental tissue and achieve a protracted therapeutic effect.

Because of the washout problem, treatment of diseased dental tissue with antibacterial compounds disclosed by the prior art has involved injection of these compounds in conjunction with polymeric delivery systems directly into the diseased tissue. For example, PCT application WO 92/07555 published May 14, 1992 discloses a drug delivery system wherein a biodegradable polymer containing the chemotherapeutic agent is heated to a physiologically compatible elevated temperature prior to injection by syringe into the diseased tissue area. As the biodegradable material cools, it hardens and remains in place. As the hardened material biodegrades, it releases the chemotherapeutic agent to the diseased tissue over a period of days.

U.S. 4,938,763 also discloses treatment of infections in body cavities using syringeable, in-situ forming biodegradable implants. In the disclosed method, an antibacterial agent is suspended or dissolved in a concentrated solution, e.g. 40-70% by weight, of a biodegradable polymer. The solution is injected by syringe into the infected body area whereupon the solvent dissipates, and the polymer solidifies to a solid implant which adheres to the surrounding tissue. The encased antibacterial agent is then released to the infected area as the implant biodegrades.

A drawback to the application of antibacterial compounds to infected dental tissue in the manner disclosed in PCT application WO 92/0755 or U.S. 4,938,763 is that injection by syringe requires the services of a dental professional to administer the antibacterial containing polymer.

There is therefore a need in the art to provide means whereby antibacterial compounds can be delivered to and retained on dental tissue for extended periods of time without salivary washout and without the need for a dental professional to administer the compound.

### SUMMARY OF THE INVENTION

The present invention provides a non-aqueous liquid oral composition containing an antibacterial compound whereby a patient can, easily and without special training, self-administer the antibacterial compound to infected dental tissue sites wherein washout of the antibacterial agent will be substantially retarded thereby enhancing the efficacy of the administered compound.

The liquid oral composition such as a mouthwash or rinse comprises a dilute nonaqueous solution of an antibacterial compound and a water insoluble, biodegradable polymer, the antibacterial agent and the biodegradable polymer being present in the solution at a combined concentration of less than about 10% by weight.

### DETAILED DESCRIPTION OF THE PREFERRED EMBODIMENTS

The present invention is based on the discovery that the presence of relatively low concentrations, e.g., about 0.1 to about 5% by weight of a biodegradable polymer in oral compositions containing antibacterial compounds substantially increases uptake of the antibacterial compound and reduces salivary washout of the compound from dental tissue.

Antibacterial compounds for use in the compositions of the present invention include any compound which provides treatment or prevention management of diseases of the oral cavity. Examples of antibacterial compounds which are particularly desirable from considerations of antiplaque effectiveness, safety and formulation include halogenated diphenyl ethers such as 2',4,4'-trichloro-2'-hydroxy-diphenyl ether (Triclosan), 2,2'-dihydroxy-5,5'-dibromo-diphenyl ether, and bis-guanido hexane compounds such as chlorhexidine and derivative compounds thereof including N¹-(4-chlorobenzyl)-N⁵-(2,4-dichlorobenzyl biguanide; p-chlorophenyl biguanide; 4-chlorobenzhydryl biguanide; 4-chlorobenzhydrylguanylurea; N-3-lauroxypropyl-N⁵-p-chlorobenzybiguanide; 1,6-di-p-chlorophenylbiguanidohexane; 1-(lauryldimethyl-ammonium)-8-(p-chlorobenzyldimethylammonium)octane dichloride; 5,6-dichloro-2-guanidinobenzimidazole; N¹-p-chlorophenyl-N⁵-laurylbiguanide and 5-amino-1,3-bis(2-ethylhexyl)-5-methylhexahydro pyrimidine.

The antibacterial compound is present in the oral composition of the present invention in an effective antiplaque amount typically about 0.01%-5% by weight, preferably about 0.1%-1% by weight of the oral composition.

The biodegradable polymer, which enhances uptake of the anti-bacterial compound to and reduces washout from oral surfaces thereby enhancing the efficacy and activity of the antibacterial compound, is employed in the oral composition in amounts effective to achieve such enhancement, such amounts being within the range of about 0.1 to about 10% by weight and preferably about 2.0% to about 4%, by weight of the oral composition. As will hereinafter be demonstrated, the presence of small amounts of the biodegradable polymer in the oral composition increases the uptake of the antibacterial compound on dental tissue in the order of 300%.

Biodegradable polymers which may be included in the nonaqueous liquid oral compositions of the present invention are solid, water insoluble polymers including polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates poly (malic acid), poly(amino acids), and copolymers, terpolymers, or combinations or mixtures of the above materials. Low molecular weight polylactic acid formed by the condensation of lactic acid and higher molecular weight poly (DL-lactide) formed by the additional polymerization of DL-lactide are preferred in the practice of the present invention.

In the preparation of the oral compositions of the present invention, the antibacterial compound and biodegradable polymer are dissolved in an organic solvent which comprises about 90 to about 98% by weight of the oral composition. Solvents which may be used to prepare the oral compositions of the present invention are non-toxic and biocompatible so as not to cause irritation of dental tissue. Examples of such solvents include N-methyl-2-pyrrolidone, 2-pyrrolidone, ethanol, propylene glycol, acetone, methyl acetate, ethyl acetate, methyl ethyl ketone, dimethylformide;, dimethyl sulfoxide, tetrahydrofuran, capropactam, and 1-dodecylazacycloheptan-2-one. Ethyl acetate and N-methyl-2-pyrrolidone are preferred as solvents in the preparation of the oral composition of the present invention.

To administer the antibacterial oral composition of the present invention to the oral cavity, the composition in the form of a mouthwash or rinse is applied by the patient onto the teeth or the oral tissue surrounding the teeth. Self-administration may also be conveniently accomplished by spraying onto the oral tissue or teeth using a periodontal gum device.

A typical gum device useful for self-administration of the oral composition of the present invention to dental tissue is described in U.S. Patent 4,617,918. A design modification of the device is described in copending, commonly assigned U.S. Design Patent Application SN 61984 filed June 8, 1987. This periodontal gum device comprises an elongated handle member terminating proximally in a flexible tip portion provided with at least one aperture for the discharge of the oral composition. The liquid oral composition of the present invention is stored in a fluid reservoir in the device from which the liquid solution is delivered to the aperture, the tip portion being configured to expedite probing contact with gingival sulcus and permit discharge of the solution into gingival sulcus.

In addition to an antibacterial compound and a biodegradable polymer, the compositions of the present invention may include a variety of optional components. Such components include but are not limited to fluoride providing compounds, surfactants, flavor oils, viscosity controlling agents and the like.

Typical fluorine providing compounds are inorganic fluoride salts such as soluble alkali metal, alkaline earth metal, and heavy metal salts, for example, sodium fluoride, potassium fluoride, ammonium fluoride, zinc fluoride, stannic fluoride, stannous fluoride, barium fluoride, sodium fluorosilicate, ammonium fluorosilicate, sodium fluorozirconate, and sodium monofluorophosphate. Alkali metal, tin fluoride and monofluorophosphates such as sodium and stannous fluoride, sodium monofluorophosphate and mixtures thereof are preferred.

When present in a liquid oral preparation such as a mouthwash or rinse, the fluorine providing compound is generally present in an amount sufficient to release up to about 0.15%, preferably about 0.001% to about 0.1% fluoride by weight of the composition.

The oral composition of the present invention may also contain flavorants and colorants which do not inactivate the antibacterial compounds. Flavorants and colorant may be incorporated in the oral compositions in minor amounts, i.e. amounts up to 2% by weight.

The present invention is further illustrated by the following example.

### EXAMPLE

This Example demonstrates the preparation and activity of a non-aqueous liquid spray composition containing triclosan and poly(DL-lactide) according to the present invention, the composition being self-administrable as a mouthwash or rinse or as a spray using the device described in U.S. Patent 4,617,918. The effect of the biodegradable polymer on the enhanced uptake of triclosan on tooth surfaces was assessed in vitro using human teeth immersed in the solutions containing the ingredients listed below:

| **Solution A** | **Solution B** |
|---|---|
| 1% Triclosan | 1% Triclosan |
| 99% Ethyl Acetate | 96% Ethyl Acetate |
| 0% Poly (DL-lactide) | 3% Poly (DL-lactide)* |

| | |
|---|---|
| * Available commercially from Birmingham Polymers, Inc. | |

Human teeth were immersed in the above solutions for 60 seconds, then rinsed with water for 5 seconds, and immersed in 95% ethanol to extract Triclosan. The ethanol extract was then analyzed for Triclosan using HPLC. The uptake of Triclosan on the teeth is a measure of triclosan activity. The results of the in vitro test are recorded in the Table below.

**TABLE**

| | **Triclosan Uptake, µg/tooth** |
|---|---|
| Solution A | 56.3 |
| Solution B | 159.0 |

The results recorded in the Table show that Triclosan uptake from the polymer containing composition (Solution B) was three-fold higher than that obtained from the composition in which the polymer was absent.

## Claims

1. An antibacterial non-aqueous liquid oral composition exhibiting increased uptake and adherence by dental tissue of antibacterial compounds incorporated therein, the composition comprising up to about 10% by weight of the total weight of the composition of an antibacterial compound, and a water insoluble biodegradable polymer, the balance being a non-aqueous organic solvent in which the antibacterial compound is dissolved or suspended.

2. The composition of claim 1 wherein the antibacterial compound is triclosan.

3. The composition of claim 1 wherein the biodegradable polymer is poly (DL-lactide).

4. The composition of claim 1 wherein the solvent is ethyl acetate.

5. The composition of claim 1 wherein the antibacterial agent is incorporated in the composition at a concentration of about 0.01 to about 1.0% by weight.

6. The composition of claim 1 wherein the biodegradable polymer is incorporated in the composition at a concentration of about 0.1 to about 10% by weight.

7. The composition of claim 1 which is in the form of a mouthwash, rinse, or spray.

8. A method for the treatment and prevention of bacterial plaque accumulation on teeth which comprises administering to the oral cavity an antibacterial non-aqueous liquid oral composition comprising up to about 10% by weight of the total weight of the composition of an antibacterial compound, and a water insoluble biodegradable polymer, the balance being a non-aqueous organic solvent in which the antibacterial compound is dissolved or suspended, the compound exhibiting increased uptake and adherence to dental tissue.

9. The method of claim 8 wherein the antibacterial compound is triclosan.

10. The method of claim 8 wherein the biodegradable polymer is poly (DL-lactide).

11. The method of claim 8 wherein the solvent is ethyl acetate.

12. The composition of claim 8 wherein the antibacterial agent is incorporated in the composition at a concentration of about 0.01 to about 1.0% by weight.

13. The composition of claim 8 wherein the biodegradable polymer is incorporated in the composition at a concentration of about 0.1 to about 10% by weight.

14. The method of claim 1 wherein the composition is administered to the oral cavity in the form of a mouthwash, rinse, or spray.
